# EUROPEAN PATENT APPLICATION

(11) **EP 1 120 122 A1**
(43) Date of publication of application: **01.08.2001**
(21) Application number: 99969680.0
(22) Date of filing: 29.09.1999
(51) Int. Cl.: A61L 27/00, A61F 2/28, C07F 9/6561

(54) **BONE REPAIR MATERIALS/ARTIFICIAL BONE COMPOSITIONS**

(30) Priority: 30.09.1998 JP 27767998
(71) Applicant: Takeda Chemical Industries, Ltd., Osaka-shi, Osaka 541-0045 (JP)
(72) Inventor: MAKINO, Haruhiko, Kawabe-gun Hyogo 666-0251 (JP); NAKAMURA, Takashi, Ukyo-ku Kyoto-shi Kyoto 615-0005 (JP); KATO, Hirohumi, Sakyo-ku Kyoto-shi Kyoto 606-8263 (JP); HOSHINO, Tetsuo, Toyono-gun Osaka 563-0105 (JP)
(74) Representative: Keller, Günter, Dr.
(86) International application number: JP9905343
(87) International publication number: WO0018444

(57) **Abstract**

Bone repair materials/artificial bone compositions prepared by adding a benzothiopyran derivative or a benzothiepin derivative to a bone repair material or an artificial bone or coating a bone repair material or an artificial bone with a benzothiopyran derivative or a benzothiepin derivative for inducing the reparation of a defective site of a natural bone and strengthening the binding of the artificial bone to the natural bone in bone graft for treating fracture, etc.

## Description

### Technical Field

The present invention relates to bone repair material and artificial bone compositions that have an effect of enhancing coaptation of bone repair material and artificial bone with natural bone.

### Background Art

Transplantation of bone has been put into practice with the highest frequency in tissue transplantation. This is clinically effected for loss of bone due to an external injury, prolonged curing or pseudarthrosis caused by a fracture of a bone or after excision of osteoncus. Bone transplantation in the living body so far has widely been employed in auto-transplantation in which the patient's own bone is used. In this bone auto-transplantation, however, there are limitations in the quantity of the patient's own bone to be used. Moreover, it requires two surgical operations for excision of bone and transplantation, which cause the patient pain.

In order to cover this disadvantage of bone auto-transplantation, artificial bones made of metal, ceramics, etc. have been developed and applied. The role of transplanted bone is in promoting osteogenesis and obtaining mechanical support. Though the mechanical support by the artificial bone is sufficient, promotion of osteogenesis cannot be expected.

In the circumstances, it has been desired to develop excellent bone repair material and artificial bone compositions which induce repair of the defective part of natural bone after bone transplantation and which can strengthen the coaptation between the natural bone and the artificial bone.

### Disclosure of Invention

In this technical background, the present inventors worked diligently to develop bone repair material and artificial bone compositions which induce repair of the defective part of natural bone after bone transplantation and which can strengthen the coaptation between the natural bone and the artificial bone. As a result, they found that it is possible to induce repair of the defective part of natural bone after bone transplantation and strengthen coaptation between the natural bone and the artificial bone by mixing or coating the bone repair material or artificial bone with a benzothiopyran or benzothiepin derivative. The inventors further investigated based on this finding, and completed the invention.

The invention provides:
(1) A composition which is produced by mixing or coating bone repair material or artificial bone with a benzothiopyran or benzothiepin derivative;
(2) A composition according to the above item (1), wherein the benzothiopyran or benzothiepin derivative is a compound having an osteogenesis or chondrogenesis promoting effect;
(3) A composition according to the above item (1), wherein the bone repair material or artificial bone is metal, ceramic material, high-molecular compound or proteinous material;
(4) A composition according to the above item (1), wherein the bone repair material or artificial bone is ceramic material;
(5) Acomposition according to the above item (1), wherein the benzothiopyran or benzothiepin derivative is formulated into a sustained release preparation;
(6) A composition according to the above item (1), wherein the benzothiopyran or benzothiepin derivative is a compound of the formula (I) : 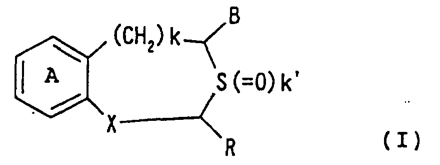 wherein the ring A is an optionally substituted benzene ring; R is a hydrogen atom or optionally substituted hydrocarbon group; B is an optionally esterified or amidated carboxy group; X is -CH(OH)- or -CO-; k is 0 or 1; and k' is 0, 1 or 2, or a salt thereof;
(7) A composition according to the above item (6), wherein the ring A is a benzene ring which may be substituted by 1 or 2 substituents selected from halogen atom, C₁₋₁₀ alkyl group, C₁₋₁₀ alkoxy group, alkylenedioxy group of the formula -O-(CH₂)ₙ-O- (where n is an integer of 1 - 3) and C₁₋₁₀ alkylthio group; R is a hydrogen atom, C₁₋₆ alkyl group or phenyl; B is a group of the formula -CON(R¹) (R²) (where R¹ is a hydrogen atom or C₁₋₁₀ alkyl group; R² is a phenyl or pheny-C₁₋₃ alkyl group which may be substituted by halogen, C₁₋₆ alkoxy, mono- or di-C₁₋₆ alkoxyphosphoryl, mono- or di-C₁₋₆ alkoxyphosphoryl-C₁₋₃ alkyl (where the dialkyl in the di-C₁₋₆ alkoxy taken together may form a C₁₋₆ alkylene group) or C₁₋₆ alkoxycarbonyl);
(8) A composition according to the above item (6), wherein the compound of the formula (I) or salt thereof is an optically active compound of the formula (II): 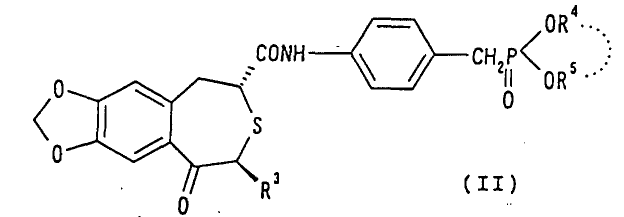 wherein R³ is a C₁₋₆ alkyl group; R⁴ and R⁵ each is a C₁₋₆ alkyl group or they taken together form a C₁₋₆ alkylene group, or a salt thereof;
(9) A composition according to the above item (8), wherein the compound of the formula (II) or salt thereof is (2*R*,4*S*)-(-)-*N*-[4-(diethoxyphosphorylmethyl)phenyl]-1,2,4,5-tetrahydro-4-methyl-7,8-methylenedioxy-5-oxo-3-benzothiepin-2-carboxamide or a salt thereof;
(10) A composition according to the above item (1), wherein the ratio of (1) the benzothiopyran or benzothiepin derivative to (2) the bone repair material or artificial bone is about 1 : 1 - 100 by weight;
(11) Use of a benzothiopyran or benzothiepin derivative for the production of the composition according to the above item (1);
(12) An enhancer for coaptation of an artificial bone with a natural bone which comprises a benzothiopyran or benzothiepin derivative as an effective component; and
(13) Use of a benzothiopyran or benzothiepin derivative for the production of the enhancer for coaptation according to the above item (12).

As for the benzothiopyran or benzothiepin derivatives used in the compositions of the invention, any derivatives may be employed as long as they can be used as medicaments. Such derivatives preferably include compounds that have osteogenesis and/or chondrogenesis-promoting effects.

As for the benzothiopyran or benzothiepin derivatives, the compounds represented by the above-mentioned formula (I) or salts thereof are particularly preferred.

In the above-mentioned formula (I), the substituent on the substituted benzene ring represented by the ring A includes, for example, a halogen atom, a nitro group, an optionally substituted alkyl group, an optionally substituted hydroxy group, an optionally substituted mercapto group, an optionally substituted amino group, an acyl group, a mono- or dialkoxyphosphoryl group, a phosphono group, an optionally substituted aryl group, an optionally substituted aralkyl group and an optionally substituted aromatic heterocyclic group; 1 to 4 of these substituents, preferably 1 or 2, which may be the same or different each other, may be substituted on the benzene ring.

The "halogen atom" includes, for example, fluorine, chlorine, bromine, iodine, and the like.

The alkyl group of the "optionally substituted alkyl group" includes, preferably, alkyl of 1 - 10 carbon atoms (e.g., methyl, ethyl, propyl, isopropyl, butyl, isobutyl, sec-butyl, tert-butyl, pentyl, isopentyl, neopentyl, hexyl, heptyl, octyl, nonyl, decyl, etc.), cycloalkyl of 3 - 7 carbon atoms (e.g., cyclopropyl, cyclobutyl, cyclohexyl, cycloheptyl, etc.), and the like; these may be substituted by 1 to 3 substituents such as, for example, halogen atom (e.g., fluorine, chlorine, bromine, iodine, etc.), hydroxy, alkoxy group of 1 - 6 carbon atoms (e.g., methoxy, ethoxy, propoxy, butoxy, hexyloxy, etc.), mono- or di-C₁₋₆ alkoxyphosphoryl group (e.g., methoxyphosphoryl, ethoxyphosphoryl, dimethoxyphosphoryl, diethoxyphosphoryl, etc.), phosphono, and the like.

The substituted alkyl group is exemplified by, for example, trifluoromethyl, trifluoroethyl, trichloromethyl, hydroxymethyl, 2-hydroxyethyl, methoxyethyl, 1-methoxyethyl, 2-methoxyethyl, 2,2-diethoxyethyl, 2-diethoxyphosphorylethyl, phosphonomethyl, and the like.

The substituted hydroxy group in the "optionally substituted hydroxy group" is exemplified by, for example, alkoxy group, alkenyloxy group, aralkyloxy group, acyloxy group, aryloxy group, and the like.

The "alkoxy group" includes, preferably, alkoxy group of 1 - 10 carbon atoms (e.g., methoxy, ethoxy, propoxy, butoxy, *tert*-butoxy, pentyloxy, hexyloxy, heptyloxy, nonyloxy, etc.), cycloalkoxy group of 4 - 6 carbon atoms (e.g., cyclobutoxy, cyclopentoxy, cyclohexyloxy, etc.). The "alkenyloxy group" includes, preferably, those of 2 - 10 carbon atoms, e.g., allyloxy, crotyloxy, 2-pentenyloxy, 3-hexenyloxy, 2-cyclopentenylmethoxy, 2-cyclohexenylmethoxy, etc. The "aralkyloxy group" includes, preferably, those of 6 - 19 carbon atoms, more preferably, (C7-C14)aryl-(C1-C4)alkyloxy (e.g., benzyloxy, phenethyloxy, etc.). The "acyloxy group" includes, preferably, alkanoyloxy group, for example, those of 2 - 10 carbon atoms (e.g., acetyloxy, propionyloxy, n-butyryloxy, hexanoyloxy, etc.). The "aryloxy group" includes, preferably, those of 6 - 14 carbon atoms (e.g., phenoxy, biphenyloxy, etc.). These groups may further be substituted by 1 - 3 of the same substituents as mentioned above such as, for example, halogen atom, hydroxy, alkoxy group of 1 - 6 carbon atoms, mono- or di-C₁₋₆ alkoxyphosphoryl group, phosphono group, and the like. The substituted hydroxy group is exemplified by, for example, trifluoromethoxy, 2,2,2-trifluoroethoxy, difluoromethoxy, 2-methoxyethoxy, 4-chlorobenzyloxy, 2-(3,4-dimethoxyphenyl) ethoxy, and the like.

The mercapto group in the "optionally substituted mercapto group" is exemplified by, for example, alkylthio group, aralkylthio group, acylthio group, and the like. The "alkylthio group" includes, preferably, alkylthio group of 1 - 10 carbon atoms (e.g., methylthio, ethylthio, propylthio, butylthio, pentylthio, hexylthio, heptylthio, nonylthio, etc.), cyclo-alkylthio group of 4 - 6 carbon atoms (e.g., cyclobutylthio, cyclopentylthio, cyclohexylthio, etc.), and the like. The "aralkylthio group" includes, preferably, those of 7 - 19 carbon atoms, more preferably, (C₆-C₁₄)aryl-(C₁-C₄)alkylthio group, e.g., benzylthio, phenethylthio, and the like. The "acylthio group" includes, preferably, alkanoylthio, for example, those of 2 - 10 carbon atoms (e.g., acetylthio, propionylthio, n-butyrylthio, hexanoylthio, etc.). These groups may further be substituted by 1 - 3 of the same substituents as mentioned above such as, for example, halogen atom, hydroxy, alkoxy group of 1 - 6 carbon atoms, mono- or di-C₁₋₆ alkoxyphosphoryl group, phosphono, and the like. The substituted mercapto group is exemplified by, for example, trifluoromethylthio, 2,2,2-trifluoroethylthio, 2-methoxyethylthio, 4-chlorobenzylthio, 3,4-dichlorobenzylthio, 4-fluorobenzylthio, 2-(3,4-dimethoxy- phenyl)ethylthio, and the like.

The substituent of the substituted amino group in the "optionally substituted amino group" includes the same alkyl group of 1 - 10 carbon atoms as mentioned above, alkenyl group of 2 - 10 carbon atoms (e.g., allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl, etc.), aryl group of 6 - 14 carbon atoms (e.g., phenyl, naphthyl, etc.), and aralkyl group of 7- 19 carbon atoms (e.g., benzyl, phenethyl, etc.); and these may be used alone or in combination of two identical or different groups. These groups may be substituted by the same halogen atom, alkoxy group of 1 - 6 carbon atoms, mono- or di-C₁₋₆ alkoxyphosphoryl group, phosphono group, and the like, as mentioned above.

The substituted amino group is exemplified by, for example, methylamino, dimethylamino, ethylamino, diethylamino, dibutyl amino, diallylamino, cyclohexylamino, phenylamino or *N*-methyl-*N*-phenylamino, *N*-methyl-*N*-(4-chlorobenzyl)amino, *N*,*N*-di(2-methoxyethyl)amino, and the like.

As for the "acyl group", an organic carboxylic acyl group or sulfonic acyl group having a hydrocarbon group of 1 - 6 carbon atoms (e.g., C₁₋₆ alkyl (e.g., methyl, ethyl, n-propyl, hexyl, etc.), phenyl, etc.) may be used. As for the "organic carboxylic acyl group", for example, formyl, alkylcarbonyl group of 1 - 10 carbon atoms (e.g., acetyl, propionyl, butyryl, valeryl, pivaloyl, hexanoyl, octanoyl, cyclobutanecarbonyl, cyclo-hexanecarbonyl, cycloheptanecarbonyl, etc.), alkenylcarbonyl group of 2 - 10 carbon atoms (e.g., crotonyl, 2-cyclohexenecarbonyl, etc.), arylcarbonyl group of 6 - 14 carbon atoms (e.g., benzoyl, etc.), aralkylcarbonyl group of 7 - 19 carbon atoms (e.g., benzylcarbonyl, benzhydrylcarbonyl, etc.), 5- or 6-membered aromatic heterocyclic carbonyl group (e.g., nicotinoyl, 4-thiazolylcarbonyl, etc.), 5- or 6-membered aromatic heterocyclic acetyl group (e.g., 3-pyridylacetyl, 4-thiazolylacetyl, etc.), and the like may be used. As for the "sulfonic acyl group having a hydrocarbon group of 1 - 6 carbon atoms", for example, methanesulfonyl, ethanesulfonyl, and the like may be used. These groups may be substituted by 1 - 3 of the same substituents as mentioned above, such as halogen atom, hydroxy, alkoxy group of 1 - 6 carbon atoms, amino, and the like. The acyl group is exemplified by, for example, trifluoroacetyl, trichloroacetyl, 4-methoxybutyryl, 3-cyclohexyloxypropionyl, 4-chlorobenzoyl, 3,4-dimethoxybenzoyl, and the like.

As for the "mono- or dialkoxyphosphoryl group", for example, mono-C₁₋₆ alkoxyphosphoryl group such as methoxyphosphoryl, ethoxyphosphoryl, propoxyphosphoryl, isopropoxyphosphoryl, butoxyphosphoryl, pentyloxyphosphoryl, hexyloxyphosphoryl, and the like, and di-C₁₋₆ alkoxyphosphoryl group such as dimethoxyphosphoryl, diethoxyphosphoryl, dipropoxyphosphoryl, diisopropoxyphosphoryl, dibutoxyphosphoryl, dipentyloxyphosphoryl, dihexyloxyphosphoryl, and the like may be used. Preferably, di-C₁₋₆ alkoxyphosphoryl group, for example, dimethoxyphosphoryl, diethoxyphosphoryl, dipropoxyphosphoryl, diisopropoxyphosphoryl, ethylenedioxyphosphoryl, dibutoxyphosphoryl, and the like may be used.

As for the aryl group in the "optionally substituted aryl group", those of 6 - 14 carbon atoms, for example, phenyl, naphthyl, anthryl, and the like, may preferably be used. These groups may be substituted by 1 - 3 of the same substituents as mentioned above, such as alkyl group of 1 - 10 carbon atoms, halogen atom, hydroxy, alkoxy group of 1 - 6 carbon atoms, and the like. The substituted aryl group is exemplified by, for example, 4-chlorophenyl, 3,4-dimethoxyphenyl, 4-cyclohexylphenyl, 5,6,7,8-tetrahydro-2-naphthyl, and the like.

As for the aralkyl group in the "optionally substituted aralkyl group", those of 7 - 19 carbon atoms, for example, benzyl, naphthylethyl, trityl, and the like may preferably be used. These groups may be substituted on their aromatic ring by 1 - 3 of the same substituents as mentioned above such as alkyl group of 1 - 10 carbon atoms, halogen atom, hydroxy, alkoxy group of 1 - 6 carbon atoms, and the like. The substituted aralkyl group is exemplified by, for example, 4-chlorobenzyl, 3,4-dimethoxybenzyl, 4-cyclohexylbenzyl, 5,6,7,8-tetrahydro-2-naphthylethyl, and the like.

As for the aromatic heterocyclic group in the "optionally substituted aromatic heterocyclic group", 5- or 6-membered ones having 1 to 4 of nitrogen atom, oxygen atom and/or sulfur atom, for example, furyl, thienyl, imidazolyl, thiazolyl, oxazolyl, thidiazolyl, and the like, may preferably be used. These groups may be substituted by 1 - 3 of the same substituents as mentioned above such as alkyl group of 1 - 10 carbon atoms, halogen atom, hydroxy, alkoxy group of 1 - 6 carbon atoms, and the like.

When two alkyl groups are placed adjacent to each other on the benzene ring A, they may be bound to each other to form an alkylene group of the formula: -(CH₂)ₘ- where m is an integer of 3 - 5 (e.g., trimethylene, tetramethylene, pentamethylene, etc.), and when two alkoxy groups are placed adjacent to each other, they may form an alkylenedioxy group of the formula: -O- (CH₂)ₙ-O- where n is an integer of 1 - 3 (e.g., methyl-enedioxy, ethylenedioxy, trimethylenedioxy, etc.). In such a case, a 5-to 7-membered ring is formed together with the carbon atoms on the benzene ring.

In the above-mentioned formula (I), R represents a hydrogen atom or optionally substituted hydrocarbon group.

As for the hydrocarbon group in the "optionally substituted hydrocarbon group" represented by R, an alkyl group (preferably, alkyl group of 1 - 10 carbon atoms, including, e.g., methyl, ethyl, propyl, isopropyl, butyl, isobutyl, sec-butyl, tert-butyl, pentyl, isopentyl, neopentyl, hexyl, etc.), the same alkenyl group as mentioned above (preferably, alkenyl group of 2 - 10 carbon atoms), as well as the same aryl group (preferably, aryl group of 6 - 14 carbon atoms), aralkyl group (preferably, aralkyl group of 7 - 19 carbon atoms), and the like as mentioned above may be used. As for the substituent on the hydrocarbon group, a 5- or 6-membered aromatic heterocylic group (e.g., furyl, thienyl, imidazolyl, thiazolyl, oxazolyl, thiadiazolyl, etc.), the same halogen atom as mentioned above, the same di-C₁₋₆ alkoxyphosphoryl group as mentioned above, phosphono, and the like may be used.

In the above-mentioned formula (I), B is an optionally esterified or amidated carboxyl group.

As for the esterified carboxyl group of the "optionally esterified carboxyl group" represented by B, for example, an alkoxycarbonyl group, preferably, C₁₋₁₀ alkoxycarbonyl group (e.g., methoxycarbonyl, ethoxycarbonyl, propoxycarbonyl, butoxycaronyl, etc.), aryloxycarbonyl group, preferably, C₆₋₁₄ aryloxycarbonyl group (e.g., phenoxycarbonyl, etc.), aralkyloxycarbonyl group, preferably, C₇₋₁₉ aralkyloxycarbonyl group (e.g., benzyloxycarbonyl, etc.), and the like may be used.

The amidated carboxyl group of the "optionally amidated carboxyl group" represented by B includes optionally substituted carbamoyl groups represented by the formula:-CON(R¹) (R²) wherein R¹ and R² each is a hydrogen atom, optionally substituted hydrocarbon group or optionally substituted 5 - 7-membered heterocyclic group.

As for the hydrocarbon group in the "optionally substituted hydrocarbon group" represented by R¹ and R², an alkyl group, preferably, alkyl group of 1 - 10 carbon atoms (e.g., methyl, ethyl, propyl, isopropyl, butyl, isobutyl, *sec*-butyl, *tert*-butyl, pentyl, isopentyl, neopentyl, hexyl, heptyl, octyl, nonyl, decyl, etc.), alkenyl group, preferably, alkenyl group of 2 - 10 carbon atoms (e.g., allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl, etc.), aryl group, preferably, aryl group of 6 - 14 carbon atoms (e.g., phenyl, naphthyl, anthryl, etc.), aralkyl group, preferably, aralkyl group of 7 - 19 carbon atoms (e.g., benzyl, naphthylethyl, trityl, etc.), and the like may be used. These hydrocarbon groups may be substituted by 1 - 3 substituents such as (i) a halogen atom (e.g., fluorine, chlorine, bromine, iodine, etc.), (ii) a hydroxy group, (iii) an alkoxy group of 1 - 6 carbon atoms (e.g., methoxy, ethoxy, propoxy, butoxy, tert-butoxy, pentyloxy, hexyloxy, etc.), (iv) an amino group optionally substituted by alkyl group of 1 - 6 carbon atoms (e.g., methyl, ethyl, propyl, isopropyl, butyl, isobutyl, *sec*-butyl, *tert*-butyl, pentyl, isopentyl, neopentyl, hexyl, etc.) (e.g., amino, methylamino, ethylamino, dimethylamino, diethylamino, dipropylamino, etc.), (v) an amino group substituted by an acyl group (e.g., alkanoyl group of 1 - 10 carbons, etc.) (e.g., acetylamino, propionylamino, benzoylamino, etc.), (vi) a carbamoyl group optionally substituted by alkyl group of 1 - 6 carbon atoms (e.g., carbamoyl, methylcarbamoyl, dimethylcarbamoyl, diethylcarbamoyl, etc.), (vii) a C₁₋₆ alkoxycarbonyl group (e.g., methoxycarbonyl, ethoxycarbonyl, propoxycarbonyl, etc.), (viii) a mono- or dialkoxyphosphoryl group (e.g., mono- or di-C₁₋₆ alkoxyphosphoryl group (e.g., dimethoxyphosphoryl, diethoxyphosphoryl, ethylenedioxyphosphoryl, etc.), etc.), (ix) a mono- or dialkoxyphosphorylalkyl group (e.g., mono- or di-C₁₋₆ alkoxyphosphoryl-C₁₋₃ alkyl group (e.g., methoxyphosphorylmethyl, ethoxyphosphorylmethyl, methoxyphosphorylethyl, ethoxyphosphorylethyl, dimethoxyphosphorylmethyl, diethoxyphosphorylmethyl, dimethoxyphosphorylethyl, diethoxyphosphorylethyl, etc.), etc.), (x) a group of the formula: 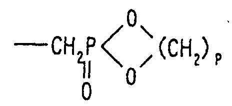 wherein p is an integer of 2 to 4,
(xi) a phosphono group, (xii) an aromatic heterocyclic group (the same as defined above), and the like.

As for the 5- to 7-membered heterocyclic group in the "optionally substituted 5- to 7-membered heterocyclic group" represented by R¹ and R², for example, a 5- to 7-membered heterocyclic group containing one sulfur atom, nitrogen atom or oxygen atom, 5- or 6-membered heterocyclic group containing 2 to 4 nitrogen atoms, or 5- or 6-membered heterocyclic group containing 1 or 2 nitrogen atoms and one sulfur atom or oxygen atom may be used. These heterocyclic groups may be condensed with a 6-membered ring containing up to 2 nitrogen atoms, benzene ring, or 5-membered ring containing one sulfur atom. As for the substituent which may be substituted on the "optionally substituted 5- to 7-membered heterocyclic group", 1 to 4 of the same substituents as those which may be substituted on the hydrocarbon group of the "optionally substituted hydrocarbon group" represented by the above-mentioned R¹ and R², may be used.

Preferred example of the 5- to 7-membered heterocyclic group represented by R¹ and R² includes, for example, 2-pyridyl, pyrimidyl, pyrazinyl, pyridazinyl, pyrazolyl, imidazolyl, thiazolyl, oxazolyl, tetrazolyl, thiadiazolyl, oxadiazolyl, triazinyl, triazolyl, thienyl, pyrrolyl, pyrrolinyl, furyl, pyrrolidinyl, benzo-thienyl, indolyl, imidazolidinyl, piperidyl, piperidino, piperadinyl, morpholinyl, morpholino, pyrido[2,3-d]pyrimidyl, benzopyranyl, 1,8-naphthylidyl, quinolyl, thieno[2,3-b]-pyridyl, and the like.

R¹ and R² in -N(R¹) (R²) taken together may form a 5- to 7-membered ring which includes morpholine, thiomorpholine, piperidine, homopiperazine, piperazine, pyrrolidine, pyrroline, pyrazoline, imidazoline, imidazolidine, thiazolidine, azepine, and the like.

The preferred substituted alkyl group in the "optionally substituted hydrocarbon group" represented by R¹ and R² is exemplified by, for example, trifluoromethyl, trifluoroethyl, difluoromethyl, trichloromethyl, 2-hydroxyethyl, 2-methoxyethyl, 2-ethoxyethyl, 2,2-dimethoxyethyl, 2,2-diethoxyethyl, 2-pyridylmethyl, 3-pyridylmethyl, 4-pyridylmethyl, 2-(2-thienyl)ethyl, 3-(3-furyl)propyl, 2-morpholinoethyl, 3-pyrrolylbutyl, 2-piperidinoethyl, 2-(*N*,*N*-dimethylamino) ethyl, 2-(*N*-methyl-*N*-ethylamino)ethyl, 2-(*N*,*N*-diisopropylamino) ethyl, 5-(*N*,*N*-dimethylamino)pentyl, *N*,*N*-dimethylcarbamoylethyl, *N*,*N*-dimethylcarbamoylpentyl, ethoxycarbonylmethyl, isopropoxycarbonylethyl, *tert*-butoxycarbonylpropyl, 2-diethoxyphosphorylethyl, 3-dipropoxyphosphorylpropyl, 4-dibutoxyphosphorylbutyl, ethylenedioxyphosphorylmethyl, 2-phosphonoethyl, 3-phosphonopropyl, and the like. The substituted aralkyl group is exemplified by, for example, 4-chlorobenzyl, 3-(2-fluorophenyl)propyl, 3-methoxybenzyl, 3,4-dimethoxyphenethyl, 4-ethylbenzyl, 4-(3-trifluoromethylphenyl)butyl, 4-acetylaminobenzyl, 4-dimethylaminophenethyl, 4-diethoxyphosphorylbenzyl, 2-(4-dipropoxyphosphorylmethylphenyl)ethyl, and the like. The substituted aryl group is exemplified by, for example, 4-chlorophenyl, 4-cyclohexylphenyl, 5,6,7,8-tetra-hydro-2-naphthyl, 3-trifluoromethylphenyl, 4-hydroxyphenyl, 3,4,5-trimethoxyphenyl, 6-methoxy-2-naphthyl, 4-(4-chlorobenzyloxy)phenyl, 3,4-methylenedioxyphenyl, 4-(2,2,2-trifluoroethoxy)phenyl, 4-propionylphenyl, 4-cyclohexanecarbonylphenyl, 4-dimethylaminophenyl, 4-benzoylaminophenyl, 4-diethoxycarbamoylphenyl, 4-tert-butoxycarbonylphenyl, 4-diethoxyphosphorylphenyl, 4-diethoxyphosphorylmethylphenyl, 4-(2-diethoxyphosphorylethyl)phenyl, 2-diethoxyphosphorylmethylphenyl, 3-diethoxyphosphorylmethylphenyl, 4-dipropoxyphosphorylphenyl, 4-(2-phosphonoethyl)phenyl, 4-phosphonomethylphenyl, 4-phosphonophenyl, and the like. The substituted 5- to 7-membered heterocyclic group is exemplified by, for example, 5-chloro-2-pyridyl, 3-methoxy-2-pyridyl, 5-methyl-2-benzothiazolyl, 5-methyl-4-phenyl-2-thiazolyl, 3-phenyl-5-isoxazolyl, 4-(4-chlorophenyl)-5-methyl-2-oxazolyl, 3-phenyl-1,2,4-thiadiazol-5-yl, 5-methyl-1,3,4-thiadiazol-2-yl, 5-acetylamino-2-primidyl, 3-methyl-2-thienyl, 4,5-dimethyl-2-furanyl, 4-methyl-2-morpholinyl, and the like. In the above-mentioned groups, the ring A is a benzene ring optionally substituted by 1 or more, preferably 1 or 2 of the same or different substituents, such as halogen atom, optionally substituted alkyl group, optionally substituted hydroxy group, optionally substituted mercapto group and/or optionally substituted amino group.

Particularly preferred ring A is a benzene ring that may be substituted by 1 or 2 of substituents, such as halogen atom, alkyl group of 1 - 10 carbon atoms (more preferably, of 1 - 5 carbon atoms), alkoxy group of 1 - 10 carbon atoms (more preferably, of 1 - 5 carbon atoms), alkylenedioxy group of the formula: -O-(CH₂)ₙ-O- where n is an integer of 1 - 3 and/or alkylthio group of 1 - 10 carbon atoms (more preferably, of 1 - 5 carbon atoms).

Especially preferred ring A is a benzene ring that is substituted at the adjacent carbon atoms by an alkylenedioxy group of the formula -O-(CH₂)ₙ-O- where n is an integer of 1 - 3.

R is preferably a hydrogen atom, C₁₋₆ alkyl group (e.g., methyl, ethyl, etc.) or phenyl.

B is preferably, for example, an alkoxycarbonyl group or a group of the formula -CON(R¹) (R²) wherein R¹ and R² each is a hydrogen atom, optionally substituted hydrocarbon group or optionally substituted 5- to 7-membered heterocyclic group.

In a preferred example of R¹ and R², R¹ is a hydrogen atom or alkyl group of 1 - 10 carbon atoms (e.g., methyl, ethyl, propyl, etc.), and R² is a phenyl or phenyl-C₁₋₃ alkyl group which may be substituted by halogen (e.g., fluorine, chlorine, bromine, etc.), C₁₋₆ alkoxy (e.g., methoxy, ethoxy, etc.), mono- or dialkoxyphosphoryl (e.g., mono- or di-C₁₋₆ alkoxyphosphoryl such as dimethoxyphosphoryl, diethoxyphosphoryl, etc.), mono- or di-alkoxyphosphorylalkyl (e.g., mono- or di-C₁₋₆ alkoxyphosphoryl-C₁₋₆ alkyl such as dimethoxyphosphorylmethyl, diethoxyphosphorylmethyl, etc.)(the dialkyl in the di-C₁₋₆ alkoxy taken together may form a C₁₋₆ alkylene group) or C₁₋₆ alkoxycarbonyl (e.g., methoxycarbonyl, ethoxycarbonyl, etc.), or an optionally phenyl-substituted 5- or 6-membered heterocyclic group containing 1 or 2 nitrogen atoms or 1 nitrogen atom and 1 sulfur atom (e.g., pyridyl, etc.).

In an especially preferred example of R¹ and R², R¹ is a hydrogen atom, and R² is a phenyl group substituted by mono- or di-C₁₋₆ alkoxyphosphoryl-C₁₋₃ alkyl (e.g., 4-diethoxyphosphorylmethylphenyl, etc.).

In the above-mentioned formula (I) , X is -CH (OH) - or -CO-, preferably, -CO-.

In the above-mentioned formula (I), k is 0 or 1, k' is 0, 1 or 2, and preferably, k is 1 and k' is 0.

In the above-mentioned formula (I), particularly preferred is, for example, an optically active benzothiepine derivative of the formula (II): 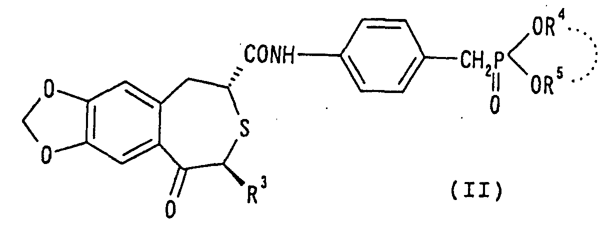 wherein R³ is C₁₋₆ alkyl group; R⁴ and R⁵ each is a C₁₋₆ alkyl group, or they taken together form a C₁₋₆ alkylene group.

In the above-mentioned formula (II), the "C₁₋₆ alkyl" represented by R³, R⁴ and R⁵ includes methyl, ethyl, propyl, isopropyl, butyl, isobutyl, sec-butyl, tert-butyl, pentyl, isopentyl, neopentyl, hexyl, and the like. Preferably, an alkyl group of 1 to 4 carbon atoms is exemplified. R⁴ and R⁵ taken together may form a C₁₋₆ alkylene group, which may be represented by the formula: 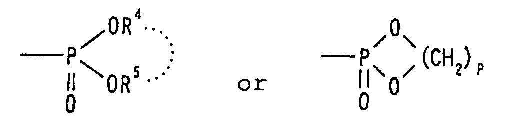 wherein p is an integer of 2 to 4.

As for R³, R⁴ and R⁵, an alkyl group of 1 to 4 carbon atoms such as methyl, ethyl, etc. is preferred.

The compounds (II) are optically active isomers with a (2*R*, 4*S*) configuration substantially containing no compound of (2*S*, 4*R*) configuration, and it is better when the optical purity is nearer to 100%.

Particularly preferred one of the compounds (II) is, for example, (2*R*,4*S*)-(-)-*N*-[4-(diethoxyphosphorylmethyl)phenyl]-1,2,4,5-tetrahydro-4-methyl-7,8-methylenedioxy-5-oxo-3-benzothiepine-2-carboxamide (hereinafter sometimes referred to as Compound A) or salts thereof. The structural formula of Compound A can be represented by the formula:

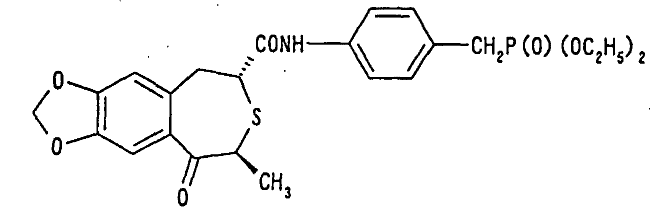

The benzothiopyran or benzothiepin derivatives used in the compositions of the invention, preferably the salts of benzothiopyran or benzothiepin derivatives having an osteogenesis or chondrogenesis promoting effect are, preferably, used as pharmacologically acceptable salts. The pharmacologically acceptable salts used include those with inorganic bases, organic bases, inorganic acids or organic acids, or with basic or acidic amino acids. The base that can form salts with the benzothiopyran or benzothiepin derivatives includes inorganic bases such as alkali metal (e.g., sodium, potassium, etc.), alkaline earth metal (e.g., calcium, magnesium, etc.), and organic bases such as trimethylamine, triethylamine, pyridine, picoline, *N*,*N*-dibenzylethylenediamine, diethanolamine, etc. The acid includes inorganic acids such as hydrochloric acid, hydrobromic acid, hydroiodic acid, phosphoric acid, nitric acid, sulfuric acid, etc., and organic acids such as formic acid, acetic acid, trifluoroacetic acid, oxalic acid, tartaric acid, fumaric acid, maleic acid, methanesulfonic acid, benzenesulfonic acid, p-toluenesulfonic acid, citric acid, etc. The basic or acidic amino acid includes, for example, arginine, lysine, aspartic acid, glutamic acid, etc.

The benzothiopyran or benzothiepin derivatives or salts thereof used in the compositions of the invention may be produced, for example, in the same process as described in JP-A 232880/1991 (European patent application publication no. (hereinafter referred to as EP-A) 376197), JP-A 364179/1992 (EP-A 460488), or JP-A 231569/1996 (EP-A 719782), or a process similar thereto or their equivalent process.

The benzothiopyran or benzothiepin derivatives used in the compositions of the invention may preferably be formulated into sustained release preparations.

The sustained release preparations include, for example, compositions containing the benzothiopyran or benzothiepin derivative and a biodegradable high molecular polymer.

The sustained release preparations containing the benzothiopyran or benzothiepin derivative and a biodegradable high molecular polymer may be produced according to a per se known method, for example, the same method as described in JP-A 263545/1997 or its equivalent method.

Preferred biodegradable high molecular polymers include those which are insoluble or practically insoluble in water and can be formed into a shape, which is degradable in the living body within a certain period of time required for therapy. Such polymers are exemplified by, for example, fatty acid polyesters (e.g., polymers or copolymers or their mixtures of one or more of α-hydroxycarboxylic acids (e.g., lactic acid, glycolic acid, 2-hydroxybutyric acid, 2-hydroxyvaleric acid, 2-hydroxy-3-methylbutyric acid, 2-hydroxycaproic acid, 2-hydroxyisocaproic acid, 2-hydroxycaprylic acid, etc.), hydroxydicarboxylic acids (e.g., malic acid, etc.), hydroxytricarboxylic acids (e.g., malic acid, etc.), lactic acid caprolactone, valerolactone, etc.), and their derivatives (e.g., polylactic acid, polyglycolic acid and polyethylene glycol block polymers, etc.), poly-α-cyano acrylate, polyalkylene oxalates (e.g, polytrimethylene oxalate, polytetramethylene oxalate, etc.), polyorthoesters, polyorthocarbonates, polycarbonates (e.g., polyethylenecarbonate, polyethylenepropylenecarbonate, etc.), polyamino acids (e.g., poly-γ-benzyl-L-glutamic acid, poly-L-alanine, poly-γ-methyl-L-glutamic acid, etc.), hyarulonic acid esters, polystyrene, polymethacrylic acid, acrylic acid-methacrylic acid copolymer, polyamino acid, dextran stearate, ethylcellulose, acetylcellulose, nitrocellulose, maleic anhydride copolymer, collagen, gelatin, fibrin, hydroxyapatite, and the like.

These biodegradable high molecular polymers may be of a single polymer of one species or copolymer or 2 or more species or their simple mixture. The type of polymerization may be in any of random, block or graft polymerization.

The preferred biodegradable high molecular polymers are, for example, aliphatic polyesters, etc. Particularly, polymers or copolymers synthesized from, for example, one or more of α-hydroxycarboxylic acids are preferred in view of their biodegradability and biocompatibility. Practically, copolymers synthesized from one or more of lactic acid, glycolic acid, 2-hydroxybutyric acid, 2-hydroxyvaleric acid, and the like, or their mixture may be used.

The biodegradable copolymer can be produced according to the known process as described in JP-A 28521/1986 or its equivalent process.

The above-mentioned α-hydroxycarboxylic acids may be any of D-isomers, L-isomers and DL-isomers, and the DL-isomers are preferred.

The above-mentioned single polymers of α-hydroxycarboxylic acids include those from lactic acid, glycolic acid, 2-hydroxybutyric acid, etc. As for the α hydroxycarboxylic acid, latic acid is preferred. The copolymers of α-hydroxy-carboxylic acids include those of glycolic acid with other type of α-hydroxycarboxylic acids, preferably, for example, lactic acid, 2-hydroxybutyric acid, and the like. Such copolymers are exemplified by, for example, lactic acid-glycolic acid copolymers, 2-hydroxybutyric acid-glycolic acid copolymers, and the like, and the lactic acid-glycolic acid copolymers are preferably used.

The weight-average molecular weight of biodegradable high molecular polymers is selected from the range of about 2,000 to about 800,000, preferably, of about 5,000 to about 200,000.

As for the single copolymers of lactic acid (hereinafter sometimes referred to as polylactic acid), those of about 5, 000 to about 100,000 of weight-average molecular weight are preferred. More preferred are those of about 6,000 to about 50,000. The polylactic acid may be synthesized according to the known process, for example, as described in JP-A 28521/1986, or its equivalent process.

In the lactic acid-glycolic acid copolymers, the compositional ratio of lactic acid to glycolic acid is preferably about 100/0 to about 50/50 (W/W), particularly about 90/10 to 50/50 (W/W). The weight-average molecular weight of lactic acid-glycolic acid copolymers is preferably about 5, 000 to about 100,000, more preferably about 8,000 to 50,000. The lactic acid-glycolic acid copolymers may be synthesized according to the known process, for example, as described in JP-A 28521/1986, or its equivalent process. The preferred copolymers include those synthesized by non-catalytic dehydrating polycondensation.

In the 2-hydroxybutyric acid-glycolic acid copolymers, those in which the content of glycolic acid is about 40 to about 70 mole%, and the residue is 2-hydroxybutyric acid are preferred. The weight-average molecular weight of 2-hydroxybutyric acid-glycolic acid copolymers is preferably about 5,000 to about 100, 000, more preferably about 8,000 to about 50,000. The 2-hydroxybutyric acid-glycolic acid copolymers may be synthesized according to the known process, for example, as described in JP-A 28521/1986. The preferred copolymers include those synthesized by non-catalytic dehydrating polycondensation.

The above-mentioned 2-hydroxybutyric acid-glycolic acid copolymers may be used further as a mixture with polylactic acid. When the 2-hydroxybutyric acid-glycolic acid copolymers is used as a mixture with polylactic acid, the ratio in the mixture is about 10/90 to about 90/10 (weight %), more preferably, about 25/75 to 75/25 (weight %).

The weight-average molecular weight means the molecular weight that is determined on gel-permeation chromatography (GPC) on the basis of polystyrene. The determination is conducted on a GPC column KF804LX2 (made by Showa Denko K.K.) and RI monitor L-3300 (Hitachi, Ltd.) using chloroform as a mobile phase.

The amount of the biodegradable high molecular polymer to be used may be altered depending on the potency of pharmacological activity of the benzothiopyran or benzothiepin derivative, for example, Compound (I) or a salt thereof, and the rate and time of drug release from the biodegradable high molecular polymer. For example, said polymer may be used in an amount of about 0.2 to 10,000 times (ratio by weight), preferably about 1 to 1,000 times (ratio by weight), and more preferably about 1 to 100 times (ratio by weight), for the physiologically active substance.

The benzothiopyran or benzothiepin derivatives, for example, Compound (I) or a salt thereof may be taken into a biodegradable high molecular compound according to a conventional manner. For example, the benzothiopyran or benzothiepin derivative, for example, Compound (I) or a salt thereof, is dispersed into a biodegradable high molecular compound to form microcapsules or microspheres, or filled into a hollow biodegradable high molecular compound which has preliminarily been formed into a certain shape. Practically, it can be achieved by means of the in-water drying method, phase separation method, spray drying method, and the like method.

The shape of the pharmaceutical preparations of the invention prepared according to the above-mentioned methods may be, for example, of fine granules, spheres, rods, needles, pellets, films, creams, and the like, but not limited to these shapes as long as the purpose is achieved.

In this description, a finely granular pharmaceutical composition is sometimes referred to as microcapsules or microspheres.

The followings describe methods for producing microcapsules.

### (1) In-water drying method (o/w method)

In this method, a solution of a biodegradable polymer in an organic solvent is first prepared. The organic solvent used in this method preferably has a boiling point of 120°C or lower. Such an organic solvent includes, for example, halogenated hydrocarbons (e.g., dichloromethane, chloroform, chloroethane, dichloroethane, trichloroethane, carbon tetrachloride, etc.), aliphatic esters (e.g., ethyl acetate, butyl acetate, etc.), ethers (e.g., ethyl ether, isopropyl ether, etc.), aromatic hydrocarbons (e.g., benzene, toluene, xylene, etc.), and the like. These solvents may be used in combination of two or more kinds in an appropriate ratio. The preferred organic solvent is dichloromethane or acetonitrile. The particularly preferred organic solvent is dichloromethane. The concentration of the biodegradable polymer dissolved in an organic solvent is generally chosen from about 0.01 to about 80% (w/w), preferably about 0.1 to about 70% (w/w), and particularly about 1 to about 60% (w/w), depending on the molecular weight of the biodegradable polymer and the type of organic solvent used, etc.

The benzothiopyran or benzothiepin derivative, for example, Compound (I) or a salt thereof is added and dissolved into the solution of the biodegradable polymer in an organic solvent thus prepared, if necessary after lyophilization or vacuum desiccation. The amount of the benzothiopyran or benzothiepin derivative, for example, Compound (I) or a salt thereof to be added is about 0.001% to about 90% (w/w), preferably about 0.01% to 80% (w/w), and particularly about 0.1% to 50% (w/w), based on the concentration of the biodegradable polymer dissolved in an organic solvent, wherein the concentration is variable depending on the type of drug used, action mechanism in cartilage destruction suppression and chondorogenesis promotion, duration of the effect, etc.

The solution of an organic solvent thus prepared is then added to an aqueous phase to form an o/w emulsion using a turbine-type mechanical stirrer or the like. The volume of the aqueous phase is normally chosen from the range of about 1 to 10,000 times, preferably about 2 to 5,000 times, and more preferably about 5 to 2,000 times, for the volume of the oil phase.

An emulsifier may be added to the aqueous phase as the above-mentioned outer phase. The emulsifier may be any one as long as it is capable of forming a stable o/w emulsion. Examples of such emulsifiers include anionic surfactants, non-ionic surfactants, polyoxyethylene castor oil derivatives, polyvinyl pyrrolidone, polyvinyl alcohol, carboxymethyl cellulose, lecithin, gelatin, hyaluronic acid, and the like. These may be used in combination as appropriate. The concentration of emulsifier in the external aqueous phase is preferably about 0.001% to about 20% (w/w), more preferably about 0.01% to about 10% (w/w), and particularly about 0.05% to about 5% (w/w).

In evaporation of the solvent from the oil phase, a conventional method can be employed. Such a method is carried out under normal or gradually reduced pressure with stirring using a propeller-type agitator, magnetic stirrer, and the like, or under control of the degree of vacuum using a rotary evaporator, and the like. Thus resulting microcapsules are collected by separation by centrifugation or filtration, and then washed with, for example, water or heptane repeatedly several times to remove the benzothiopyran or benzothiepin derivative, for example, Compound (I) or a salt thereof, the emulsifier, etc. adhering on the surface of microcapsules. Then, the microcapsules are again dispersed in distilled water, etc. and lyophilized. In order to prevent cohesion among particles during washing, it is appropriate to add an anticoagulant (for example, water-soluble sugars such as mannitol, lactose, glucose, starch (e.g., corn starch), etc., amino acids such as glycine, alanine, etc., proteins such as gelatin, fibrin, collagen, etc., and the like).

In the above-mentioned o/w method, the microcapsules may also be produced by dispersing the benzothiopyran or benzothiepin derivative, for example, Compound (I) or a salt thereof into a solution of the biodegradable polymer in an organic solvent, that is, the s/o/w method.

### (2) In-water drying method (w/o/w method)

In this method, the benzothiopyran or benzothiepin derivative, for example, Compound (I) or a salt thereof is first dissolved or dispersed in water so as to be the concentration specified above. If necessary, a drug-retaining substance such as a protein (e.g., gelatin, etc.), seaweed (e.g., agar, etc.), polysaccharide (e.g., alginic acid, etc.), synthetic high-molecular substance (e.g., polyvinyl alcohol, etc.), basic amino acid (e.g., arginine, lysine) or the like is dissolved or suspended therein to give an internal aqueous phase. In order to keep the stability and solubility of the benzothiopyran or benzothiepin derivative, for example, Compound (I) or a salt thereof, it is also appropriate to add a pH regulator into the internal aqueous phase. Such a pH regulator includes organic acids such as acetic acid, oxalic acid, citric acid, etc., inorganic acids such as carbonic acid, phosphoric acid, etc., alkali metal hydroxides such as sodium hydroxide, etc., basic amino acids such as arginine, lysine, etc., and salts thereof (e.g., salts with organic acids such as acetic acid, oxalic acid, citric acid, etc., or salts with inorganic acids such as carbonic acid, phosphoric acid, hydrochloric acid, etc.). As a stabilizer for the benzothiopyran or benzothiepin derivative, for example, Compound (I) or a salt thereof, there may be added a protein (e.g., albumin, gelatin, etc.), starch derivative (e.g., dextrin, pullulan, etc.), organic acid (e.g., citric acid, etc.), alkali metal salt of ethylenediaminetetraacetic acid (e.g., sodium ethylenediaminetetraacetate), alkali metal hydrogen sulfite (e.g., sodium hydrogen sulfite, etc.), synthetic high-molecular substance (e.g., polyethylene glycol, etc.) or the like. In addition, a usually used preservative such as paraoxybenzoic acid ester (e.g., methyl paraben, propyl paraben, etc.), benzyl alcohol, chlorobutanol, thimerosal, and the like may also be added. The amount of the benzothiopyran or benzothiepin derivative, for example, Compound (I) or a salt thereof to be added is about 0.001% to about 90% (w/w), preferably about 0.01% to 80% (w/w), and particularly about 0.1% to 50% (w/w), as a concentration in the internal aqueous phase, wherein the concentration is variable depending on the type of drug used, action mechanism in cartilage destruction suppression and chondorogenesis promotion, duration of the effect, etc.

Thus resulting internal aqueous phase is added to a solution (oil phase) containing the biodegradable high molecilar polymer, followed by emulsification to yield a w/o emulsion. This emulsification is achieved by a known dispersing method such as an intermittent shaking method, a method using a mixer such as a propeller-type agitator or a turbine-type agitator, a colloidal mill method, a homogenizer method and an ultrasonication method. The above-mentioned solution (oil phase) containing the biodegradable high molecular polymer is a solution prepared by dissolving a biodegradable high molecular substance in an organic solvent. Such a solvent includes those of which the boiling point is about 120°C or lower and which is immiscible with water. For example, halogenated hydrocarbons (e.g., dichloromethane, chloroform, chloroethane, dichloroethane, trichloroethane, carbon tetrachloride, etc.), aliphatic esters (e.g., ethyl acetate, butyl acetate, etc.), ethers (e.g., ethyl ether, isopropyl ether, etc.), aromatic hydrocarbons (e.g., benzene, toluene, xylene, etc.), and the like may be used. These solvents may be used in combination of two or more kinds in an appropriate ratio. In order to prevent cohesion among particles during washing, it is appropriate to add an anticoagulant (for example, water-soluble sugars such as mannitol, lactose, glucose, starch (e.g., corn starch), etc., amino acids such as glycine, alanine, etc., proteins such as gelatin, fibrin, collagen, etc., and the like).

Thus produced w/o emulsion is then added to an aqueous phase to give a w/o/w emulsion, from which the oil phase solvent is evaporated to yield microcapsules. Practically, this operation may be carried out in the same manner as in the above item (1).

### (3) Phase separation method

In this method, a coacervation agent is slowly added to the above-mentioned w/o emulsion under stirring to precipitate and solidify the biodegradable high molecular polymer. The coacervation agent includes compounds of high molecular type, mineral oil type or vegetable oil type which are miscible with a solvent for the biodegradable high molecular polymer and in which the polymer for formation of microcapsules is insoluble. Such compounds are exemplified by, for example, silicon oil, vegetable oils and fats (e.g., sesame oil, soybean oil, corn oil, cotton seed oil, coconut oil, linseed oil, etc.), mineral oils, hydrocarbons (e.g., n-hexane, n-heptane, etc.), and the like. These may be used in combination of two or more kinds.

Thus resulting microcapsules are collected by filtration, then repeatedly washed with heptane, etc. to remove the coacervation agent. Then, removal of the released drug and elimination of the solvent are conducted according to the same manner as in the in-water drying method.

In order to prevent cohesion among particles during washing, it is appropriate to add an anticoagulant (for example, water- soluble sugars such as mannitol, lactose, glucose, starch (e.g., corn starch), etc., amino acids such as glycine, alanine, etc., proteins such as gelatin, fibrin, collagen, etc., and the like).

### (4) Spray drying method

When the microcapsules are produced in this method, the above-mentioned w/o emulsion is sprayed through a nozzle into a drying chamber of a spray dryer (spray-drying apparatus) to volatilize the organic solvent and moisture contained in the fine droplets within a very short period of time yielding microcapsules. Said nozzle includes a two-fluid nozzle, pressure nozzle, rotary disc nozzle, and the like. In this stage, in order to prevent cohesion of microcapsules caused concurrently with spraying of the w/o emulsion, it is also effective to spray an aqueous solution of the above-mentioned anticoagulant through another nozzle. The microcapsules thus obtained, if required, may be warmed under reduced pressure to attain more complete removal of the water and solvent contained therein.

The particle size of the microcapsules, for example, the average particle size is in the range of about 0.1 to about 300 µm, preferably about 1 - 150 µm, more preferably about 2 - 100 µm.

In preparing microcapsules as a sterile preparation, a method conducing the entire production process under a sterile condition, a method of sterilization by gamma-ray, a method using an antiseptic as additive, and the like may be employed, but the method is not limited to them.

In addition to the above-mentioned microcapsules, a biodegradable high molecular polymer in which the benzothiopyran or benzothiepin derivative, for example, Compound (I) or a salt thereof is dispersed, may be dissolved and formulated into spheres, rods, needles, pellets, and the like.

Moreover, it is also possible to pulverize the biodegradable high molecular polymer in which the benzothiopyran or benzothiepin derivative, e.g., Compound (I) or a salt thereof is dispersed, to give an appropriate particle size according to a method using a turbo counter jet mill or an ultrasonic jet mill, for example, as described in JP-A 234656/1994. Practically, the benzothiopyran or benzothiepin derivative, for example, Compound (I) or a salt thereof is dissolved in an organic solvent containing the biodegradable high molecular polymer. The mixture is then dried in vacuo to give a solid solution, which is then roughly crushed and sieved. This is then subjected to removal of the solvent and pulverized with an ultrasonic jet mill to give particles of a controlled size.

Pharmaceutical preparations for the above-mentioned sustained release are preferably made into microcapsules.

The bone repair material or artificial bone used in the compositions of the invention includes the known bio-material for plastic surgery, for example, metallic material, ceramic material, high-molecular material, proteinous material or their composite material. Particularly, the metallic material, ceramic material, and high-molecular material are preferred. The bone repair material may also be so-called bone supplementation material.

The above-mentioned metallic material includes, for example, titanium, titanium alloy, stainless steel, cobalt-chromium alloy, and the like.

The above-mentioned ceramic material includes, for example, bioinert ceramics such as alumina ceramic, single crystal alumina ceramic, zirconia ceramic, etc., and bioactive ceramics such as bioglass (Hench et al., Biomed. Master. Symp., 2, 117(1972)), hydroxyapatite (Aoki et al., Ceramics, 10, 469 (1975)), apatite-wollastonite(AW)-glass (Bull. Inst. Chem. Res. Kyoto Uni., 60, 260 (1982)), TCP ceramics (Ca₃(PO₄)₂), etc.

The above-mentioned high molecular material preferably includes, for example, poly(methyl methacrylate) (PMMA), high density polyethylene (HDP), silicone rubber, Teflon, polyester, polylactate, PVA hydrogel, and the like.

The above-mentioned proteinous material includes, for example, collagen, fibrin, chitin, chitosan, and the like.

As for the bone repair material or artificial bone used in the composition of the invention, the ceramics material is preferred, particularly, apatite-wollastonite(AW)-glass is preferred in view of its high mechanical strength and superior bioactivity.

The compositions of the invention are applicable in treatment of bone defect or as bone coapting agent for bone transplantation in mammals (e.g., human, mouse, rat, rabbit, dog, cat, bovine, equine, porcine, etc.). The compositions of the invention are lesser toxic and can be used safely.

The compositions of the invention may be prepared by mixing or coating the above-mentioned bone repair material or artificial bone with the benzothiopyran or benzothiepin derivative (hereinafter sometimes referred to as the present compound), for example, Compound (I) or a salt thereof or their sustained release preparation.

The mixing is effected so that, when a bone repair agent or artificial bone is filled or transplanted in an intended part of the bone defect, the present compound or a salt thereof is released toward the vital tissue of defective part in the local part. Practically, said compound is mixed with, kneaded with, adhered to, or contained in the bone repair agent or artificial bone. For example, when the artificial bone is used, those of which the surface has a surface property and surface structure suitable for the above-mentioned mixing are preferably used. In a typical example, the surface is made porous. Formation of the porous surface may be achieved according to a known method, for example, a method in which granules made from a homogeneous material is combined into 2 layers to make a gap between the granules, a method in which a continuing metal fiber is bound at random to form 2 layers, etc.

Said coating is achieved by applying or coating the present compound or a salt thereof on the bone repair material or artificial bone in a conventional manner. In applying or coating, the present compound or a salt thereof is dispersed in a proper dispersing agent, binder, diluent, etc. (e.g., collagen, physiological saline, citric acid solution, acetic acid solution, hydroxyapatite, fibrin, or a mixture of them), and applied to the bone repair material or artificial bone. Alternatively, the bone repair material or artificial bone may be immersed in the above dispersed mixture. Thereafter, they are dried.

When the compositions of the invention are applied to mammals, the following methods are employed.
(1) The benzothiopyran or benzothiepin derivative or a salt thereof or their sustained release preparation is mixed with or applied to the bone repair material or artificial bone to give a composition of the invention, which is filled into the bone defect part.
(2) The benzothiopyran or benzothiepin derivative or a salt thereof or their sustained release preparation is injected around the bone repair material or artificial bone which has previously been filled in a local part, so that they are mixed or covered therewith in the living body.
(3) The benzothiopyran or benzothiepin derivative or a salt thereof or their sustained release preparation is injected to around the bone repair material or artificial bone which has been filled during a surgical operation or injected to a gap formed between the above material or bone and natural bone so that they are mixed or covered therewith.

In the above-mentioned items (2) and (3), the benzothiopyran or benzothiepin derivative or a salt thereof or their sustained release preparation acts as a coaptation enhancer which promotes and enhances coaptation of the bone repair material or artificial bone with natural bone.

In applying the injection as mentioned in the above item (2), the benzothiopyran or benzothiepin derivative or a salt thereof or their sustained release preparation is dissolved or suspended or emulsified in a sterile aqueous solution or oily solution, which is usually used in a conventional injection preparation, to yield an aqueous solution.

The aqueous solution for injection preparation includes physiological saline, isotonic solution, and the like. If required, a suitable emulsifier, for example, carboxymethyl cellulose sodium, non-ionic surfactant, etc., may be used in combination. The oily solution includes vegetable oils such as sesame oil, soybean oil, corn oil, and the like.

In preparing the injections, an aqueous suspension is prepared together with a dispersing agent (e.g., surfactant such as Tween 80, HCO-60; polysaccharide such as carboxymethyl cellulose, sodium alginate, hyaluronic acid; polysorbate, etc.), preservative (e.g., methylparaben, propylparaben, etc.), isotonic agent (e.g., sodium chloride, mannitol, sorbitol, glucose, etc.), buffering agent (e.g., calcium carbonate, etc.), pH-regulator (e.g., sodium phosphate, potassium phosphate, etc.), to give a practical injection preparation.

The practically utilizable injections may also be prepared by dispersing the present compound or a salt thereof into the above-mentioned vegetable oil or its mixture with a phospholipid such as lecithin or into a middle chain fatty acid triglyceride (e.g., Miglyol 812, etc.). A solubilizing agent such as benzyl benozate, benzyl alcohol, etc. may be used in combination.

Thus prepared solution for injection is usually filled in suitable ampoules.

For applying the above-mentioned item (3), the present compound or a salt thereof is dispersed in a proper dispersing agent, binder, diluent, etc. (e.g., collagen, physiological saline, citric acid solution, acetic acid solution, hydroxyapatite, fibrin, or a mixture of them), and applied to or permeated in the bone repair material or artificial bone to be attached thereto or contained therein.

In applying the compositions of the invention, the present compound or a salt thereof is mixed with a physiologically acceptable dispersing agent, binder, diluent, and/or other agent effective for osteoanagenesis (e.g., calcium), and may be filled into a gap between a bone defect part and a fixing agent of artificial bone that is implanted in the bone defect part of a host.

The ratio of (1) the benzothiopyran or benzothiepin derivative to (2) the bone repair material or artificial bone contained in the composition of the invention is about 1 : 1 to 100 by weight, more preferably about 1 : 1 to 30 by weight, wherein the composition comprises the bone repair material or artificial bone mixed or coated with the benzothiopyran or benzothiepin derivative.

The benzothiopyran or benzothiepin derivatives may be used in an effective dose enough to firmly fix the transplanted artificial bone on the bone defect part of a host. Such a dose is, for example, about 1 - 1,000 mg/cm² (per area of the above-mentioned filled part), preferably about 10 - 100 mg/cm².

The daily dose of the composition of the invention for an adult (weighing 50 kg) as a benzothiopyran or benzothiepin derivative is about 0.1 - 100 mg, preferably about 1 - 50 mg.

### Best Mode for Carrying Out the Invention

### Example

The following Reference Examples and working Examples describe the present invention in more detail, but the invention is not limited to these examples.

Compound A used in the following examples is (2*R*,4*S*)-(-)-*N*-[4-(diethoxyphosphorylmethyl)phenyl]-1,2,4,5-tetrahydro-4-methyl-7,8-methylenedioxy-5-oxo-3-benzothiepin-2-carboxamide, which was produced according to the method as described in JP-A 231569/1996.

### Reference Example 1

According to the following recipe as shown in Table 1, a solution (hereinafter sometimes referred to as "Solution A") of a lactic acid-glycolic acid copolymer (hereinafter sometimes referred to as "PLGA"; the molar ratio (mole %) of lactic acid-glycolic acid and the weight average molecular weight determined by GPC are as shown in Table 1) and a lactic acid homopolymer (hereinafter sometimes referred to as "PLA") dissolved in dichloromethane is prepared. In the same manner, a solution is prepared from 0.1 g of Compound A and 1.0 ml of dichloromethane (hereinafter sometimes referred to as "Solution B"). Solution A is mixed with Solution B homogeneously, and poured into 0.1% polyvinyl alcohol (EG-40, The Nippon Synthetic Chemical Ind. Co., Ltd.) aqueous solution (PVA solution) pre-regulated at 15°C of Table 1. The mixture is stirred with a turbine-type homogenizer at 7, 000 rpm to give an O/W emulsion. The O/W emulsion is stirred at room temperature for 3 hours to evaporate dichloromethane. The oil phase is solidified and collected by centrifugation at 2,000 rpm with a centrifuge (05PR-22, Hitachi, Ltd.). This is again dispersed into distilled water and centrifuged to wash the released drug. The recovered microcapsules are re-dispersed into a small amount of distilled water and lyophilized.

**Table 1**

| Microcapsule No. | Solution A | | | | Dichloromethane (ml) | PVA Volume (ml) |
|---|---|---|---|---|---|---|
| | Polymer | Molar Ratio (%) | M_{w}* | Ploymer Weight (g) | | |
| 1 | PLGA | 75/25 | 10500 | 2.4 | 2.5 | 400 |
| 2 | PLGA | 75/25 | 6500 | 2.4 | 1.0 | 400 |
| 3 | PLGA | 75/25 | 17100 | 2.4 | 1.5 | 400 |
| 4 | PLA | 100/0 | 12000 | 2.4 | 2.0 | 800 |
| 5 | PLGA | 90/10 | 20000 | 2.4 | 2.0 | 400 |
| 6 | PLGA | 85/15 | 12100 | 2.4 | 2.0 | 800 |

| | | | | | | |
|---|---|---|---|---|---|---|
| * Weight-average molecular weight | | | | | | |

### Reference Example 2

PLGA (0.55 g) (lactic acid-glycolic acid: molar ratio 85 : 15; weight-average molecular weight 14,000) and Compound A (4.45 g) were dissolved in 8 mL of dichloromethane to yield an oil layer. This solution was quickly poured into 800 mL of 0.1% PVA solution to yïeld an o/w emulsion. This was further stirred at room temperature for 3 hours to evaporate dichloromethane. The resulting solidified microcapsules were collected by centrifugation and washed with distilled water. Mannitol (0.6 g) was added, and the mixture was dispersed to suspend in a small amount of water and lyophilized. The resulting microcapsules were further dried at 42°C in vacuo to completely remove the residual dichloromethane.

### Reference Example 3

In a centrifuge tube is placed about 8 g of lactic acid-valerolactone copolymer (PLV 2500ML, Taki Chemical Co., Ltd.) or glycolic acid-caprolactone copolymer (PGC 2500MG, Taki Chemical Co., Ltd.). After heating at about 50°C on a water bath, about 80 mg of Compound A was added and homogeneously dispersed to give an ointment-like preparation. This is stored in a cold place.

### Reference Example 4

Microcapsules No. 1 (500 mg) prepared in Reference Example 1 was dispersed homogeneously into two tubes containing a fibrinogen solution for Tseal (Nippon Zoki Pharmaceutical Co., Ltd.), to which is then slowly added two tubes of thrombin for Tseal. The mixture is then quickly taken up into a plastic syringe for injection and allowed to stand at 37°C for 30 minutes to solidify. The solidified content is then pushed out from the tip of the syringe and cut with a razor into pellets of about 200 µl volume.

### Example 1

Microcapsules (100 mg) (containing 10 mg of Compound A) produced according to the method as described in Reference Example 2 was mixed with 0.9 g glass ceramic containing apatite-wollastonite (hereinafter abbreviated to "AW-GC") of 15 X 10 X 2 mm average size. The mixture was implanted in the end of the tiba shaft of one leg in a Japanese white rabbit.

In the tiba shaft end of the other leg as control was implanted a mixture of 0.9 g of the same AW-GC plate as mentioned above with 100 mg of microcapsules (containing no Compound A) prepared from PLGA.

After implantation, the rabbits were divided into 3 groups. The first group was killed after 4 weeks, the second group after 8 weeks, and the third group after 16 weeks, respectively. A detaching test (peeling off) was carried out to determine the coaptation strength between the bone and the plate. The result is shown in Table 2.

**Table 2**

| coaptation strength by a detaching test (Kgf mean ± SD) | | | |
|---|---|---|---|
| | After 4 weeks | After 8 weeks | After 16 weeks |
| AW-GC plate containing Compound A | 5.266 ± 0.982* | 8.039 ± 1.109* | 10.985 ± 1.423* |
| | | | |
| Control | 2.23 ± 0.562 | 5.589 ± 0.649 | 7.191 ± 1.027 |

| | | | |
|---|---|---|---|
| * p < 0.005 vs Control in the Student t-test | | | |

As seen from the result of the detaching test as shown in Table 2, the groups in which Compound A was used in combination showed a significantly greater coaptation strength than the control group at the respective time points of 4 weeks, 8 weeks and 16 weeks (p < 0.005).

From these results, it is understood that the coaptation strength between the bone and the AW-GC plate is enhanced mechanically by the composition of the invention.

### Industrial Applicability

The bone repair material and artificial bone compositions of the invention have, for example, an effect of enhancing coaptation of a bone repair material or artificial bone with natural bone and make a repair material, artificial bone or artificial joint fixed rapidly and firmly to natural bone.

The bone repair material and artificial bone compositions of the invention, accordingly, are useful as bone repairing agents for bone defect parts, and as bone-coaptation agents in transplantation of artificial bone or in surgical operations for substitution of artificial joints.

## Claims

1. A composition which is produced by mixing or coating bone repair material or artificial bone with a benzothiopyran or benzothiepin derivative.

2. A composition according to Claim 1, wherein the benzothiopyran or benzothiepin derivative is a compound having an osteogenesis or chondrogenesis promoting effect.

3. A composition according to Claim 1, wherein the bone repair material or artificial bone is metal, ceramic material, high molecular compound or proteinous material.

4. A composition according to Claim 1, wherein the bone repair material or artificial bone is ceramic material.

5. A composition according to Claim 1, wherein the benzothiopyran or benzothiepin derivative is formulated into a sustained release preparation.

6. A composition according to Claim 1, wherein the benzothiopyran or benzothiepin derivative is a compound of the formula (I): 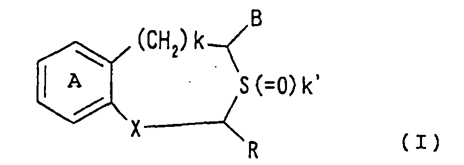 wherein the ring A is an optionally substituted benzene ring; R is a hydrogen atom or an optionally substituted hydrocarbon group; B is an optionally esterified or amidated carboxy group; X is -CH(OH)- or -CO-; k is 0 or 1; and k' is 0, 1 or 2, or a salt thereof.

7. A composition according to Claim 6, wherein the ring A is a benzene ring which may be substituted by 1 or 2 substituents selected from a halogen atom, C₁₋₁₀ alkyl group, C₁₋₁₀ alkoxy group, alkylenedioxy group of the formula -O-(CH₂)ₙ-O- where n is an integer of 1 - 3 and C₁₋₁₀ alkylthio group; R is a hydrogen atom, C₁₋₆ alkyl group or phenyl; B is a group of the formula -CON(R¹) (R²) where R¹ is a hydrogen atom or C₁₋₁₀ alkyl group; R² is a phenyl or pheny-C₁₋₃ alkyl group which may be substituted with halogen, C₁₋₆ alkoxy, mono- or di-C₁₋₆ alkoxyphosphoryl, mono- or di-C₁₋₆ alkoxyphosphoryl-C₁₋₃ alkyl where the dialkyl in the di-C₁₋₆ alkoxy taken together may form a C₁₋₆ alkylene group, or C₁₋₆ alkoxycarbonyl.

8. A composition according to Claim 6, wherein the compound of the formula (I) or a salt thereof is an optically active compound of the formula (II): 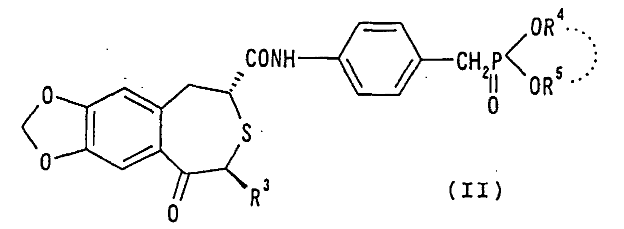 wherein R³ is a C_{1-∈} alkyl group; R⁴ and R⁵ each is a C₁₋₆ alkyl group or they taken together form a C₁₋₆ alkylene group, or a salt thereof.

9. A composition according to Claim 8, wherein the compound of the formula (II) or salt thereof is (2*R*,4*S*)-(-)-N-[4-(diethoxyphosphorylmethyl)phenyl]-1,2,4,5-tetrahydro-4-methyl-7,8-methylenedioxy-5-oxo-3-benzothiepin-2-carboxamide or a salt thereof.

10. A composition according to Claim 1, wherein the ratio of (1) the benzothiopyran or benzothiepin derivative to (2) the bone repair material or artificial bone is about 1 : 1 - 100 by weight.

11. Use of a benzothiopyran or benzothiepin derivative for the production of the composition according to Claim 1.

12. An enhancer for coaptation of an artificial bone with a natural bone which comprises a benzothiopyran or benzothiepin derivative as an effective component.

13. Use of a benzothiopyran or benzothiepin derivative for the production of the enhancer for coaptation according to Claim
